# EUROPEAN PATENT SPECIFICATION

(11) **EP 4 442 696 B1**
(45) Date of publication and mention of the grant of the patent: **02.12.2026**
(21) Application number: 21969824.8
(22) Date of filing: 31.12.2021
(51) Int. Cl.: C07K 7/06, A61K 8/00, A61K 38/08, A61P 17/00, A61Q 19/00

(54) **HEXAPEPTIDE, COSMETIC COMPOSITION OR PHARMACEUTICAL COMPOSITION CONTAINING SAME, AND USE OF HEXAPEPTIDE**
HEXAPEPTID, KOSMETISCHE ZUSAMMENSETZUNG ODER PHARMAZEUTISCHE ZUSAMMENSETZUNG DAMIT UND VERWENDUNG VON HEXAPEPTID
HEXAPEPTIDE, COMPOSITION COSMÉTIQUE OU COMPOSITION PHARMACEUTIQUE LE CONTENANT ET UTILISATION D'HEXAPEPTIDE

(43) Date of publication of application: 09.10.2024
(73) Proprietor: Shenzhen Winkey Technology Co., Ltd, Shenzhen, Guangdong 518000 (CN)
(72) Inventor: DING, Wenfeng, Shenzhen, Guangdong 518100 (CN); PENG, Yan, Shenzhen, Guangdong 518100 (CN); CHEN, Xue, Shenzhen, Guangdong 518100 (CN); ZHAO, Wenhao, Shenzhen, Guangdong 518100 (CN); SUN, Xinlin, Shenzhen, Guangdong 518100 (CN); HUANG, Chunqing, Shenzhen, Guangdong 518100 (CN); GUAN, Fuyi, Shenzhen, Guangdong 518100 (CN); XIAO, Yu, Shenzhen, Guangdong 518100 (CN)
(74) Representative: Bayramoglu et al.
(86) International application number: PCT/CN2021/143978
(87) International publication number: WO 2023/123479

(56) References cited:
- WO-A1-2013/064583
- WO-A1-2020/161683
- CN-A- 104 023 737
- US-B2- 9 725 483

## Description

### TECHNICAL FIELD

The present invention relates to a hexapeptide, a cosmetic composition or pharmaceutical composition containing the hexapeptide and use thereof.

### BACKGROUND

As the largest organ of the human body, the skin can protect internal organs from ultraviolet radiation, physical and chemical damage and microbial invasion, prevent the loss of water, electrolytes and nutrients in *vivo*, and participate in maintaining the normal physiological functions of the body. However, UV radiation, physical and chemical damage, and microbial invasion can damage the barrier function of the skin to a certain extent, causing damage to skin tissue cells and leading to the inflammatory response accompanied with acute responses such as redness, pain, and burning. On the other hand, if the barrier function of the human skin is weak, the skin will be extremely sensitive to external stimuli, and any slight stimulation may cause facial telangiectasia and hyperplasia, leading to skin stress congestion and blood stasis, as well as skin pores and capillary necrosis accompanied with occurrence of local responses such as allergies and ulcers. The fusion or remaining of fur and toxins on the human skin causes chronic inflammatory and allergic skin diseases.

In the process of inflammatory response, there are some inflammatory factors such as interleukins IL-1α, IL-6, IL-8 and tumor necrosis factor TNF-α that carry out intercellular information transmission. As a proinflammatory cytokine, IL-6 is synthesized by fibroblasts, endothelial cells, monocytes, keratinocytes and T lymphocytes, and released. In the meanwhile, its synthesis is strongly stimulated by Toll-like receptors and IL-1, and it has a higher concentration in the process of inflammatory response. IL-6 participates in the inflammatory response and is released early in the damage response, inducing macrophages, keratinocytes, endothelial cells, and stromal cells in the tissue to release proinflammatory cytokines. Since the increase of IL-6 is earlier than that of other cytokines, CRP and PCT, it can be used to assist in the early diagnosis of acute infections. As a cytokine involved in the systemic inflammatory response, TNF-α has the function of modulating the immune system and plays an important role in the process of inflammation, cell proliferation, differentiation and apoptosis. In *vitro*, TNF-α has many biological effects of killing transformed cells, stimulating granulocytes and fibroblasts, and destroying endothelial cells; in *vivo*, TNF-α as an endogenous mediator of inflammation, immunity and host defense functions plays a crucial role in many pathological conditions. At the same time, TNF-α can act independently and in conjunction with other factors that affect the different body functions as a whole.

Protease-activated receptor-2 (PAR-2) is a cell surface receptor of trypsin, which can be abundantly expressed in all types of skin cells, especially keratinocytes, and plays an important role in maintaining the dynamic balance of the skin barrier function. PAR-2 contains 7 transmembrane helices, of which the N-terminus is located extracellularly and has the protease cleavage site of the corresponding receptor; the C-terminus is located intracellularly and is related to receptor desensitization and signal transduction. Trypsin cleaves the N-terminal amino acid residue of PAR-2 between Arg36 and Ser37 to expose the tethered ligands SLIGKV (human) or SLIGRL (mouse). The tethered ligands bind to the second extracellular loop-2 of PAR-2, thereby activating receptors and inducing the release of inflammatory mediators (IL-6 and IL-8) and sensitizing the transient receptor potential vanilloid subtype 1 (TRPV-1), so that the inflammatory polypeptides calcitonin gene-related peptides (CGRPs) and Substance P are released and the inflammatory response is aggravated.

Peptide compounds for cosmetic and/or pharmaceutical use in skin-related applications have been described in the prior art. For example, WO 2013/064583 A1 discloses peptides and cosmetic and pharmaceutical uses thereof, including use in the treatment of inflammation and in the stimulation and/or care of the skin barrier function.

US 9,725,483 B2 also discloses peptide compounds, preparation processes, cosmetic and/or pharmaceutical compositions containing them, and their use in medicine, particularly in the treatment and/or prevention of pain, inflammation, itching, pigmentation disorders and angiogenic skin disorders, and in processes of treatment and/or care of the skin and/or mucous membranes.

WO 2020/161683 A1 further discloses peptide compounds useful for the treatment and/or prevention of symptoms of skin aging, in particular for the treatment and/or prevention of skin wrinkles, the treatment and/or prevention of a sagging appearance of the skin, and/or the reduction and/or prevention of facial asymmetry.

The inventors studied PAR-2 and tethered ligands thereof, and unexpectedly found that some polypeptides can inhibit the production of inflammatory factors, especially the production of inflammatory factors IL-6 and TNF-α, while promoting cell proliferation and repairing the skin barrier.

### SUMMARY

The present invention aims to provide a polypeptide that has anti-inflammatory activity. These peptides and cosmetic compositions or pharmaceutical compositions containing these peptides exert anti-inflammatory activity by down-regulating the expressions of IL-6 and TNF-α. They can be used in the field of cosmetics or medicines for improving skin inflammation and repairing the skin barrier.

In this regard, the present invention provides a peptide represented by Formula (I), or a stereoisomer thereof, or a mixture of stereoisomers thereof, or a cosmetically acceptable salt thereof, or a pharmaceutically acceptable salt thereof,

R₁-Phe-Phe-Trp-Phe-X₁-X₂-R₂ (I)

in Formula (I),
X₁-X₂ is -Lys-Val-;
R₁ is selected from H or R₃-CO-, wherein R₃ is selected from substituted or unsubstituted alkyl, substituted or unsubstituted alkenyl;
R₂ is selected from -NR₄R₅ or -OR₄, wherein each R₄ and R₅ is independently selected from H, substituted or unsubstituted alkyl, substituted or unsubstituted alkenyl;
the alkyl refers to a saturated aliphatic linear or branched chain alkyl having 1-24 carbon atoms (optionally having 1-16 carbon atoms; optionally having 1-14 carbon atoms; optionally having 1-12 carbon atoms; or optionally having 1, 2, 3, 4, 5, or 6 carbon atoms); optionally selected from methyl, ethyl, isopropyl, isobutyl, *tert*-butyl, pentyl, hexyl, heptyl, octyl, decyl, dodecyl, tetradecyl, hexadecyl, octadecyl, 2-ethylhexyl, 2-methylbutyl, or 5-methylhexyl;
the alkenyl refers to a linear or branched chain alkenyl having 2-24 carbon atoms (optionally having 2-16 carbon atoms; optionally having 2-14 carbon atoms; optionally having 2-12 carbon atoms; optionally having 2, 3, 4, 5, or 6 carbon atoms); the alkenyl has one or more carbon-carbon double bonds, optionally having 1, 2, or 3 conjugated or non-conjugated carbon-carbon double bonds; the alkenyl is bonded to the rest portions of a molecule through a single bond, and is optionally selected from vinyl, oleyl, or linoleyl;
optionally, the substituent in the "substituted alkyl" or "substituted alkenyl" is selected from C₁-C₄ alkyl; hydroxyl; C₁-C₄ alkoxy; amino; C₁-C₄ aminoalkyl; C₁-C₄ carbonyloxy; C₁-C₄ oxycarbonyl; halogen (such as fluorine, chlorine, bromine, and iodine); cyano; nitro; azide; C₁-C₄ alkylsulfonyl; thiol; C₁-C₄ alkylthio; C₆-C₃₀ aryloxy such as phenoxy; - NR_{b}(C=NR_{b})NR_{b}R_{c}, wherein R_{b} and R_{c} are independently selected from H, C₁-C₄ alkyl, C₂-C₄ alkenyl, C₂-C₄ alkynyl, C₃-C₁₀ cycloalkyl, C₆-C₁₈ aryl, C₇-C₁₇ aralkyl, a heterocyclic group having 3 to 10 members, or an amino protecting group.

Optionally, R₁ is selected from H, acetyl, tert-butyryl, hexanoyl, 2-methylhexanoyl, capryloyl, decanoyl, lauroyl, myristoyl, palmitoyl, stearoyl, oleoyl or linoleoyl;
optionally, R₁ is selected from H, acetyl, lauroyl, myristoyl or palmitoyl;
optionally, R₁ is H, acetyl or palmitoyl.

Optionally, R₄ and R₅ are each independently selected from H, methyl, ethyl, hexyl, dodecyl or hexadecyl;
optionally, R₄ is H and R₅ is selected from H, methyl, ethyl, hexyl, dodecyl or hexadecyl;
optionally, R₂ is -OH or -NH₂.

The peptide represented by Formula (I), or a stereoisomer thereof, or a mixture of stereoisomers thereof, or a cosmetically acceptable salt thereof, or a pharmaceutically acceptable salt thereof, is selected from the following peptides (1)-(6):
(1) H-Phe-Phe-Trp-Phe-Lys-Val-OH;
(2) Ac-Phe-Phe-Trp-Phe-Lys-Val-OH;
(3) Palm-Phe-Phe-Trp-Phe-Lys-Val-OH;
(4) H-Phe-Phe-Trp-Phe-Lys-Val-NH₂;
(5) Ac-Phe-Phe-Trp-Phe-Lys-Val-NH₂;
(6) Palm-Phe-Phe-Trp-Phe-Lys-Val-NH₂.

The peptide represented by Formula (I) of the present invention may be present as a stereoisomer or a mixture of stereoisomers thereof; for example, these amino acids comprised therein may have an L- or D-configuration, or be each independently racemic. Thus, it is possible to obtain isomeric mixtures as well as racemic or diastereomeric mixtures, or pure diastereomers or enantiomers, depending on the number of asymmetric carbons and what isomers or isomeric mixtures are present. Preferred structures of the peptides represented by Formula (I) of the present invention are pure isomers, i.e., enantiomers or diastereomers.

For example, when -Phe- is mentioned in the present invention, it should be understood that -Phe- is selected from -L-Phe-, -D-Phe-, or a mixture of both, and is racemic or non-racemic. The preparation methods described in this document enable a person skilled in the art to obtain each stereoisomer of the peptide of the present invention by selecting an amino acid having the correct configuration.

These peptides of the present invention may be compounds in which at least one hydrogen atom is substituted with deuterium or tritium.

The term "a cosmetically acceptable salt or pharmaceutically acceptable salt" refers to a salt approved for use in animals, and more specifically in humans, including a metal salt of the peptide represented by Formula (I), wherein the metal includes, but is not limited to, lithium, sodium, potassium, calcium, magnesium, manganese, copper, zinc or aluminum, etc.; a salt formed from the peptide represented by Formula (I) and an inorganic acid or an organic acid, wherein the organic acid includes, but is not limited to, acetic acid, citric acid, lactic acid, malonic acid, maleic acid, tartaric acid, fumaric acid, benzoic acid, aspartic acid, glutamic acid, succinic acid, oleic acid, trifluoroacetic acid, oxalic acid, pamoic acid, or gluconic acid, etc.; the inorganic acid includes, but is not limited to, hydrochloric acid, sulfuric acid, boric acid or carbonic acid.

Another aspect of the present invention provides a cosmetic or pharmaceutical composition, comprising an effective amount of the peptide represented by Formula (I), or a stereoisomer thereof, or a mixture of stereoisomers thereof, or a cosmetically acceptable salt thereof, or a pharmaceutically acceptable salt thereof, and at least one excipient and optionally a cosmetically or pharmaceutically acceptable adjuvant.

Optionally, the adjuvant is selected from a collagen synthesis stimulator, an agent that modulates PGC-1α synthesis, an agent that modulates the activity of PPARγ, an agent that increases or decreases triglyceride content in adipocytes, an agent that stimulates or delays adipocyte differentiation, a lipolytic agent or an agent that stimulates lipolysis, a fat clearing agent, an adipogenic agent, an inhibitor against acetylcholine receptor aggregation, an agent that inhibits muscle contraction, an anticholinergic agent, an elastase inhibitor, a matrix metalloproteinase inhibitor, a melanin synthesis stimulator or inhibitor, a whitening or decoloring agent, a pigmentation promoter, a self-tanning agent, an antiaging agent, a NO-synthase inhibitor, a 5α-reductase inhibitor, an inhibitor against lysyl hydroxylase and/or prolyl hydroxylase, an antioxidant, a free radical scavenger and/or an anti-air pollution agent, a reactive carbonyl species scavenger, an anti-glycation agent, an antihistamine, an antiviral agent, an antiparasitic agent, an emulsifier, an emollient, an organic solvent, a liquid propellant, a skin conditioner, a humectant, a moisture retaining substance, an alpha-hydroxy acid, a beta-hydroxy acid, a moisturizer, an epidermal hydrolase, a vitamin, an amino acid, a protein, a pigment or colorant, a dye, a biopolymer, a gelling polymer, a thickener, a surfactant, a softener, an adhesive, a preservative, an anti-wrinkle agent, an agent that can reduce or treat under-eye bags, an exfoliant, a cuticle stripping agent, a keratolytic agent, an antimicrobial, an antifungal, a fungistat, a bactericide, a bacteriostat, an agent that stimulates the synthesis of dermal or epidermal macromolecules and/or inhibits or prevents the degradation thereof, an agent that stimulates elastin synthesis, an agent that stimulates decorin synthesis, an agent that stimulates laminin synthesis, an agent that stimulates defensin synthesis, an agent that stimulates chaperone protein synthesis, an agent that stimulates cAMP synthesis, a heat shock protein, an agent that stimulates HSP70 synthesis, an agent that stimulates the synthesis of a heat shock protein, an agent that stimulates the synthesis of hyaluronic acid, an agent that stimulates fibronectin synthesis, an agent that stimulates deacetylase synthesis, an agent that stimulates the synthesis of a lipid and a stratum corneum component, ceramide, a fatty acid, an agent that inhibits collagen degradation, an agent that inhibits elastin degradation, an agent that inhibits serine protease, an agent that stimulates fibroblast proliferation, an agent that stimulates keratinocyte proliferation, an agent that stimulates adipocyte proliferation, an agent that stimulates melanocyte proliferation, an agent that stimulates keratinocyte differentiation, an agent that inhibits acetylcholinesterase, a skin relaxant, an agent that stimulates glycosaminoglycan synthesis, an anti-hyperkeratosis agent, a comedolytic agent, an antipsoriatic agent, an anti-dermatitis agent, an anti-eczema agent, a DNA repair agent, a DNA protecting agent, a stabilizer, an antipruritic agent, an agent for the treatment and/or care of sensitive skin, a hardening agent, a tightening agent, a restructuring agent, an anti-stretching agent, an adhesive, an agent that modulates sebum production, an antiperspirant, an agent that stimulates healing, an agent that assists healing, an agent that stimulates re-epithelialization, an agent that assists re-epithelialization, a cytokine growth factor, a sedative, an anti-inflammatory agent, an anesthetic agent, an agent acting on capillary circulation and/or microcirculation, an agent that stimulates angiogenesis, an agent that inhibits vascular permeability, a venotonic agent, an agent acting on cell metabolism, an agent for improving dermis-epidermis connection, an agent that induces hair growth, a hair growth inhibiting or retarding agent, a fragrance, a chelating agent, a plant extract, an essential oil, a marine extract, a reagent derived from a biological fermentation process, an inorganic salt, a cell extract, a sunscreen, and an effective organic or inorganic photoprotectant against A and/or B UV rays, or mixtures thereof.

Optionally, a preparation of the cosmetic or pharmaceutical composition is selected from a cream, an oil, milk, a balm, a foam, a lotion, a gel, a liniment, a sera, a soap, a shampoo, a hair conditioner, a serum, an ointment, a mousse, a pomade, a powder, a bar, a pencil, a spray, an aerosol, a capsule, a tablet, a granule, a chewing gum, a solution, a suspension, an emulsion, a syrup, an elixir, a polysaccharide film, a jelly or gelatin;
optionally, the capsule includes a soft capsule, a hard capsule, optionally a gelatin capsule;
optionally, the tablet includes a sugar-coated tablet.

The peptides of the present invention have variable solubilities in water depending on the nature of their sequences or any potential modifications in the N-terminus and/or C-terminus. The peptide of the present invention may therefore be incorporated into a composition via an aqueous solution, and those that are insoluble in water may be soluble in cosmetically or pharmaceutically acceptable conventional solvents, including but not limited to ethanol, propanol, isopropanol, propylene glycol, glycerin, butylene glycol or polyethylene glycol or any combination thereof.

The cosmetically or pharmaceutically effective amount of the peptide of the present invention to be administered, as well as doses thereof, will depend on many factors including age, the state of a patient, the severity of a disorder or disease, the route and frequency of administration, and specific properties of the peptide to be used.

"A cosmetically or pharmaceutically effective amount" refers to an amount of one or more peptides of the present invention that is nontoxic but sufficient to provide the desired effects. The peptide of the present invention is used in the cosmetic or pharmaceutical composition of the present invention at a cosmetically or pharmaceutically effective concentration to obtain the desired effects. In one preferred form, relative to the total weight of the composition, the concentration is between 0.00000001% (by weight) and 20% (by weight), preferably between 0.000001% (by weight) and 15% (by weight), more preferably between 0.0001% (by weight) and 10% (by weight), and even more preferably between 0.0001% (by weight) and 5% (by weight).

Another aspect of the present invention provides a cosmetically or pharmaceutically acceptable delivery system or sustained release system, to achieve better penetration of the active ingredient and/or to improve pharmacokinetic and pharmacodynamic properties thereof, which comprises an effective amount of the peptide represented by Formula (I), or a stereoisomer thereof, or a mixture of stereoisomers thereof, or a cosmetically acceptable salt thereof, or a pharmaceutically acceptable salt thereof, or the cosmetic or pharmaceutical composition above.

The term "delivery system" refers to a diluent, adjuvant, excipient or carrier administered with the peptide of the invention, selected from water, oil or surfactants, including those petroleum-derived, animal-derived, plant-derived, or synthesis-derived. Examples include but are not limited to peanut oil, soybean oil, mineral oil, sesame oil, castor oil, polysorbate, sorbitan ester, ether sulfate, sulfate, betaine, glucoside, maltoside, fatty alcohol, nonoxynol, poloxamer, polyoxyethylene, macrogol, dextrose, glycerin, digitonin and the like. Diluents that can be used in the different delivery systems in which the peptide of the present invention can be administered are known to those of ordinary skill in the art.

The term "sustained release" is used in the conventional sense to refer to a delivery system that provides a gradual release of a compound over a period of time, and preferably, but not necessarily, a relatively constant level of compound release over the entire period of time.

Examples of the delivery system or sustained release system include a liposome, an oleosome, a nonionic surfactant liposome vesicle, an ethosome, a millicapsule, a microcapsule, a nanocapsule, a nanostructured lipid carrier, a sponge, a cyclodextrin, a lipoid vesicle, a micelle, a millisphere, a microsphere, a nanosphere, a liposphere, a microemulsion, a nanoemulsion, a milliparticle, a microparticle or a nanoparticle. The preferred delivery system or sustained release system is a liposome and a microemulsion, more preferably a water-in-oil microemulsion having an internal structure of reverse micelle.

The sustained release system can be prepared by methods known in the art and can be administered, for example, by topical or transdermal administration, including adhesive patches, non-adherent patches, sealing patches, and microelectronic patches; or by systemic administration, such as and without limitation, oral or parenteral routes, including nasal, rectal, subcutaneous implantation or injection, or direct implantation or injection into specific body parts. Preferably a relatively constant amount of these peptides of the present invention should be released. The amount of a peptide comprised in the sustained release system will depend, for example, on the site where the composition is to be administered, the release kinetics and duration of the peptide of the present invention, and the nature of the condition, disorder and/or disease to be treated and/or cared for.

Another aspect of the present invention provides the peptide represented by Formula (I), or a stereoisomer thereof, or a mixture of stereoisomers thereof, or a cosmetically acceptable salt thereof, or a pharmaceutically acceptable salt thereof, or the cosmetic or pharmaceutical composition above, or the cosmetically or pharmaceutically acceptable delivery system or sustained release system above for use in the preparation of a cosmetic composition or a pharmaceutical composition for anti-inflammation.

Another aspect of the present invention provides the peptide represented by Formula (I), or a stereoisomer thereof, or a mixture of stereoisomers thereof, or a cosmetically acceptable salt thereof, or a pharmaceutically acceptable salt thereof, or the cosmetic or pharmaceutical composition above, or the cosmetically or pharmaceutically acceptable delivery system or sustained release system above for use in the preparation of a cosmetic composition or a pharmaceutical composition for skin barrier repair.

In order to facilitate understanding of the present invention, the meanings of some terms and expressions used in the present invention are explained as follows.

In the present invention, the term "skin" should be understood to be the layers which comprise it, from the uppermost layer or stratum corneum to the lowermost layer or subcutaneous tissue, both inclusive. These layers are composed of different types of cells such as keratinocytes, fibroblasts, melanocytes and/or adipocytes, among others. In the present invention, the term "skin" includes the scalp.

In the description, the abbreviations used for amino acids follow the rules specified by the IUPAC-IUB Commission of Biochemical Nomenclature in the European Journal of Biochemistry (Eur. J. Biochem. 1984, 138:9-37).

Therefore, for example, Lys represents NH₂-CH(CH₂CH₂CH₂CH₂NH₂)-COOH, Lys- represents NH₂-CH(CH₂CH₂CH₂CH₂NH₂)-CO-, -Lys represents -NH-CH(CH₂CH₂CH₂CH₂NH₂)-COOH, and -Lys- represents -NH-CH(CH₂CH₂CH₂CH₂NH₂)-CO-. Thus, a hyphen representing a peptide bond removes OH in amino acid 1-carboxyl (herein represented in the conventional non-ionized form) when located to the right of the symbol, and removes H in amino acid 2-amino when located to the left of the symbol; both modifications can be applied to the same symbol (see Table 1).

**Table 1 Structures of amino acid residues and one-letter and three-letter abbreviations thereof**

| Name/Abbreviation | Residue | Name/Abbreviation | Residue |
|---|---|---|---|
| Phenylalanyl | 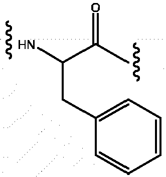 | Lysyl | 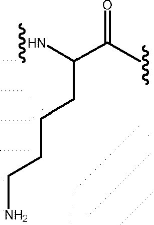 |
| -Phe- | | -Lys- | |
| F | | K | |
| Tryptophanyl | 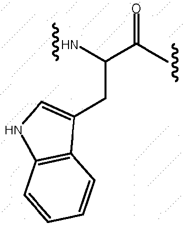 | Valyl | 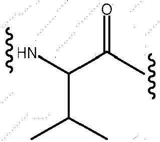 |
| -Trp- | | -Val- | |
| W | | V | |
| Histidyl | 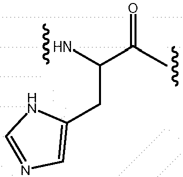 | Glycyl | 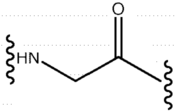 |
| -His- | | -Gly- | |
| H | | G | |
| Leucyl | 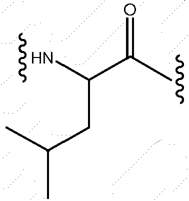 | | |
| -Leu- | | | |
| L | | | |

The abbreviation "Ac-" is used to represent acetyl (CH₃-CO-) in the present invention, and the abbreviation "Palm-" is used to represent palmitoyl (CH₃-(CH₂)₁₄-CO-).

The beneficial effects that the present invention achieves over the prior art include:

The peptides of the present invention are obtained by artificial design, easy to synthesize, can promote the proliferation of keratinocytes, down-regulate the expressions of IL-6 and TNF-α, improve the skin barrier function, and can be used in the field of cosmetics or medicines for anti-inflammation and skin barrier repair.

### BRIEF DESCRIPTION OF THE DRAWINGS

FIG. 1 is a graph showing the effect results of peptide (5) according to the present invention and comparative peptides on the HaCaT cell viability. * indicates a statistical difference between the drug group and the control group, p<0.05 (n=4).
FIG. 2 is a graph showing the effect results of peptide (5) according to the present invention and comparative peptides at 100 ppm on TNF-α expression. ### indicates an extremely significant difference between the stimulation group with LPS alone and the control group, p<0.001 (n=4). *** indicates an extremely significant difference between the drug group and the stimulation group with LPS alone, p<0.001 (n=4).
FIG. 3 is a graph showing the effect results of peptide (5) according to the present invention and comparative peptides at 100 ppm on IL-6 expression. ### indicates an extremely significant difference between the stimulation group with LPS alone and the control group, p<0.001 (n=4). * indicates a statistical difference between the drug group and the stimulation group with LPS alone, p<0.05 (n=4). *** indicates an extremely significant difference between the drug group and the stimulation group with LPS alone, p<0.001 (n=4).
FIG. 4 is a graph showing the improvement results of the TEWL value detected when using peptide (5) according to the present invention and a comparative peptide for 3 days after SDS stimulation.
FIG. 5 relates to photos showing the skin imaging results tested on the 1st and 3rd days after SDS stimulation.
FIG. 6 is a graph showing the results of the erythema index tested on the 1st and 3rd days after SDS stimulation.
FIG. 7 is a graph showing the improvement results of the erythema index peptide (5) according to the present invention and a comparative peptide for 3 days after SDS stimulation. * indicates a statistical difference between the polypeptide group and the SDS group, p<0.05.

### DETAILED DESCRIPTION OF THE EMBODIMENTS

In order to better understand the present invention, the invention will be described in detail below in conjunction with examples and drawings. However, it should be understood that these examples and drawings are only used for illustration purposes, and are not intended to limit the scope of the present invention.

### Abbreviations

The abbreviations used for amino acids follow the rules specified by the Biochemical Nomenclature Commission of IUPAC-IUB in Eur. J. Biochem. (1984) 138:9-37 and J. Chem (1989) 264:633-673.

Amide Resin: a starting resin for polypeptide synthesis (cross-linking degree 1%, substitution degree 1.42 mmol/g); Fmoc-Linker: 4-[(2,4-dimethoxyphenyl)(Fmoc-amino)methyl]phenoxyacetic acid; Ac₂O: acetic anhydride; DMF: N,N-dimethylformamide; DIPEA: diisopropylethylamine; DIC: diisopropylcarbodiimide; piperidine: piperidine; HOBt: 1-hydroxybenzotriazole; TFA: trifluoroacetic acid; TIS: triisopropylsilane; EDT: 1,2-ethanedithiol; Phe: phenylalanine; Trp: tryptophan; His: histidine; Val: valine; Lys: lysine; Leu: leucine; Gly: glycine; Fmoc: 9-fluorenylmethoxycarbonyl; Boc: tert-butoxycarbonyl; Trt: trityl.

### EXAMPLE 1

### Preparation of Fmoc-Phe-Phe-Trp(Boc)-Phe-Lys(Boc)-Val-Linker-Amide Resin

### 1.1. Preparation of Fmoc-Linker-Amide Resin

5 g Amide Resin was weighed and added to a solid-phase synthesis reaction column, and swollen with DMF. The resin was washed and the solvent was removed.

8.3 g Fmoc-Linker and 2.517 g HOBt were weighed in a dry conical flask. After DMF was used for dissolution, the solution was cooled in an ice-water bath for 2 min. 3.187 mL DIC was added for activation for 3 min while avoiding water vapor.

The activated Fmoc-Linker was added to the swollen resin to react for 3.5 h, and then the reaction solution was removed. The resin was washed and the solvent was removed.

3.8 g Ac₂O, 0.956 g DIPEA, and 20 mL DMF were further added for capping for 1.5 h. The resin was washed and the solvent was removed.

### 1.2 De-Fmoc

Fmoc-Linker-Amide Resin was Fmoc-deprotected twice using 20% piperidine/DMF, each time for 10 min. A sample was taken for the K-test, showing a color of dark blue. The resin was washed with DMF six times, and the solvent was removed.

### 1.3 Feeding reaction

6.3 g Fmoc-Val-OH and 3.011 g HOBt were weighed and added into a dry conical flask. DMF was added to dissolve the mixture, and the solution was sealed and placed in a refrigerator at -18°C for 30 min. 3.8 mL DIC was added for activation for 3 min while avoiding water vapor. The activated amino acid was added to the deprotected resin to react for 2 h. The reaction solution was removed. The K-test showed that the resin was colorless and transparent, indicating that the reaction was complete.

The N-terminal Fmoc group was deprotected, and in the presence of 3.011 g HOBt and 3.8 mL DIC, 8.7 g of the activated Fmoc-Lys(Boc)-OH was coupled to the peptidyl resin using DMF as a solvent. The reaction lasted 2 h. The resins were then washed and repeatedly subjected to the deprotection treatment of the Fmoc group so as to couple to the next amino acid. In each coupling, in the presence of 3.011 g HOBt and 3.8 mL DIC, and DMF as solvent, the resin was sequentially coupled to 7.2 g Fmoc-Phe-OH; 9.8 g Fmoc-Trp(Boc)-OH; 7.2 g Fmoc-Phe-OH and subsequently 7.2 g Fmoc-Phe-OH. After the completion of the reaction, the resin was washed and the solvent was removed.

### EXAMPLE 2

### Preparation of Ac-Phe-Phe-Trp(Boc)-Phe-Lys(Boc)-Val-Linker-Amide Resin

The N-terminal Fmoc group of the peptidyl resin obtained in Example 1 was deprotected using 20% piperidine/DMF twice, each time for 10 min. A sample was taken for the K-test, showing a color of dark blue. The resin was washed with DMF six times, and the solvent was removed.

3.8 g Ac₂O, 1.2mL DIPEA, and 20 mL DMF were added to react for 1.5 h. The resin was washed and the solvent was removed.

### EXAMPLE 3 Preparation of Ac-Phe-Phe-Trp-Phe-Lys-Val-NH₂

### 3.1 Preparation of a lysis buffer

38.5 mL TFA, 1.05 mL TIS, 1.05 mL water and 1.05 mL EDT were measured and added to a conical flask and mixed evenly with magnetically stirring to afford a lysis buffer, which was sealed and placed in a -18°C refrigerator for subsequent use.

Isopropyl ether was placed in a -18°C refrigerator for subsequent use.

### 3.2 Cleavage and sedimentation

10 g of the Ac-Phe-Phe-Trp(Boc)-Phe-Lys(Boc)-Val-Linker-Amide Resin was weighed and added to a round-bottom flask, the above-mentioned frozen lysis buffer was added, and the mixture was stirred and reacted for 2.5 h. The reaction product was filtered. The filtrate was collected and concentrated to 1/4 of the volume for later use.

320 mL of pre-frozen isopropyl ether was added to a 5 L gas collection bottle. Under stirring conditions, the above-mentioned concentrated solution for standby was slowly added into the gas collection bottle. A large amount of solids precipitated, and the upper layer became clear. There was an obvious solid-liquid stratification.

The supernatant liquid was poured off, and the remaining liquid was poured into a 250 mL centrifuge bottle for centrifugation at 4000 rpm for 2 min. The supernatant was removed. Isopropyl ether was added with stirring for washing, and centrifuged six times until the pH value was 3-4.

The solid obtained by the above centrifugation was dried under vacuum. 5.5 g of a crude peptide (purity 76%) was obtained.

### 3.3 Purification

5.5 g of the crude peptide was weighed and dissolved in 200 mL of solvents (methanol: acetic acid: water = 8:1:1), to afford a clear and transparent solution by filtering with a 0.22 µm microporous membrane. The solution was subjected to reversed-phase HPLC purification. The purification gradient is as follows:

| Time (min) | Flow rate (mL/min) | A% (acetonitrile) | B% (0.1% acetic acid aqueous solution) |
|---|---|---|---|
| 0 | 40 | 15 | 85 |
| 12 | 40 | 18 | 82 |
| 40 | 40 | 45 | 55 |
| 54 | 40 | 60 | 40 |
| 57 | 40 | 75 | 25 |
| 68 | 40 | 75 | 25 |

The filtered sample was injected and purified. Fractions were collected, concentrated and freeze-dried to afford the peptide (5) Ac-Phe-Phe-Trp-Phe-Lys-Val-NH₂ having a purity of 99.027%.

### EXAMPLE 4

Other compounds can be prepared in similar processes as above.

The molecular weight of the obtained peptides was determined by ESI-MS. The test results of some compounds are shown in Table 2 below.

**Table 2 Determination of molecular weight by mass spectrometry**

| No. | Sequence | Molecular weight mass spectrometry analysis results |
|---|---|---|
| (5) | Ac-Phe-Phe-Trp-Phe-Lys-Val-NH₂ | 913.53 |
| (11) not according to the present invention | Ac-Phe-Phe-Trp-Phe-His-Leu-NH₂ | 936.54 |
| (17) not according to the present invention | Ac-Phe-Phe-Trp-Phe-His-Gly-NH₂ | 880.38 |

### EXAMPLE 5 Cell viability assay

### 5.1 Reagents and materials

Phosphate-buffered saline (PBS) (Gibco), thiazolyl blue (MTT) (Sigma), dimethyl sulfoxide (DMSO) (Sigma), a high glucose culture medium (DMEM) (Gibco), and fetal bovine serum (Gibco).

### 5.2 Instruments

A microplate reader (MD, USA), a CO₂ incubator (Shanghai Yiheng), and an ultra-clean workbench (Suzhou Purification).

### 5.3 Cell lines

Human keratinocytes (HaCaT) were purchased from the Kunming Cell Bank of China Center for Type Culture Collection under the Chinese Academy of Sciences.

### 5.4 Samples to be tested

Drug groups:
The peptide Ac-Phe-Phe-Trp-Phe-His-Val-NH₂ (hereinafter referred to as the reference peptide) at a test concentration of 1 ppm, 10 ppm, 50 ppm, and 100 ppm respectively;
the peptide (5) at a test concentration of 1 ppm, 10 ppm, 50 ppm, and 100 ppm, respectively;
the peptide (11), which is a comparative peptide not according to the present invention, at a test concentration of 1 ppm, 10 ppm, 50 ppm, and 100 ppm, respectively;
the peptide (17), which is a comparative peptide not according to the present invention, at a test concentration of 1 ppm, 10 ppm, 50 ppm, and 100 ppm, respectively.

Control group:
PBS blank control.

### 5.5 Experimental protocol

The frozen HaCaT cells were cultured and passaged at a ratio of 1:2 to about the 5th generation, and the cells in better growth were selected as experiment subjects.

The cells were inoculated in a 96-well plate at 2000 cells/well. After the cells adhered to the wall, samples of the drug groups and the control group were added according to the multiple dilution method, supplemented with a culture medium to 200 µL, and incubated in a 5% CO₂ incubator at 37°C for 72 h.

Then 22 µL 5 mg/ml MTT was added to each well and the incubation was continued in the 5% CO₂ incubator at 37°C for 4 h. The original solution was removed and 150 µL/well DMSO was added. After 5 min, the microplate reader was used to read the reference OD values at 490 nm and 630 nm wavelengths.

### 5.6 Results

The MTT assay is a method of detecting cell survival and growth. The control group is taken as a reference value. Four dosage concentrations were set from high to low for each sample, and cell viability assays were carried out on HaCaT cells. The measured OD values are directly proportional to cell viability.

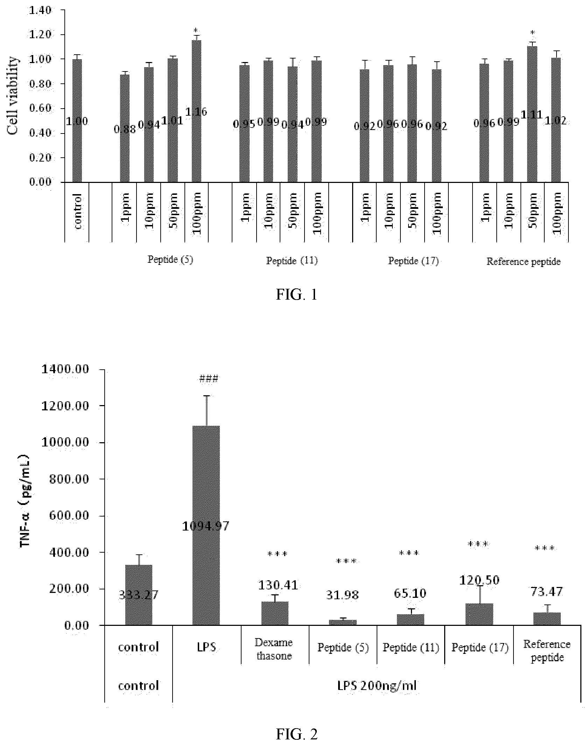

FIG. 1 is a graph showing the effect results of peptide (5) according to the present invention and comparative peptides on the HaCaT cell viability. The results showed that compared with the control group, the peptide (5), the peptide (11) as a comparative peptide, the peptide (17) as a comparative peptide and the reference peptide at 100 ppm had no toxicity to HaCaT cells. The reference peptide and the peptide (5) produced significant proliferation effects on cells at concentrations of 50 ppm and 100 ppm, respectively.

### EXAMPLE 6 Anti-inflammatory cell assay

### 6.1 Reagents and materials

Fetal bovine serum, DMEM culture medium, penicillin, streptomycin, an IL-6 ELISA kit and a TNF-α ELISA kit.

### 6.2 Instruments

A microplate reader, a constant temperature CO₂ incubator, and a thermostat.

### 6.3 Cell lines

Mouse monocyte macrophages (RAW 264.7 cells).

### 6.4 Samples to be tested

Drug groups:
100 ppm of the reference peptide, 100 ppm of the peptide (5), 100 ppm of the peptide (11) as a comparative peptide not according to the present invention, and 100 ppm of the peptide (17) as a comparative peptide not according to the present invention; the peptides were dissolved with a PBS solution, respectively.

Control group:
PBS blank control.

### 6.5 Experimental protocol

The frozen RAW 264.7 cells were cultured and passaged at a ratio of 1:2 to about the 5th generation, and the cells in better growth were selected as experiment subjects.

A cell suspension was diluted properly and inoculated on a 6-well plate at 100,000 cells/well. When the cells were about 80% confluent, an LPS-stimulated inflammation model was established. A blank control group (control), a LPS group, a dexamethasone group (DXM), and polypeptide groups were set. The blank control group was supplemented with the culture medium to 1000 µL; in the LPS group, 200 ng/mL LPS was added, and supplemented with the culture medium to 1000 µL; in the dexamethasone group, 200 ng/mL LPS and 50 ng/mL dexamethasone were added, and supplemented with the culture medium to 1000 µL; in the polypeptide groups, 200 ng/mL LPS and a polypeptide were added, and supplemented with culture medium to 1000 µL (the final test concentration of the polypeptide was 100 ppm). The above groups were further incubated in a 5% CO₂ incubator at 37°C for 48 h. The solutions were centrifuged at 3000 rpm for 15 min, in each of which a cell supernatant was collected to obtain a sample liquid. Operations were performed according to the instructions of the IL-6 and TNF-α ELISA kits.

### 6.6 Results

Inflammatory factors such as TNF-α and IL-6 can cause the body's immune overresponse, leading to skin ulcers, redness and other problems. Therefore, inhibiting inflammatory factors such as TNF-α and IL-6 secreted by monocytes in the skin can achieve anti-inflammatory and repairing effects. An inflammation model was established by stimulating RAW 264.7 cells with 200 ng/mL LPS to investigate the anti-inflammatory and repairing effects of the peptides of the present invention.

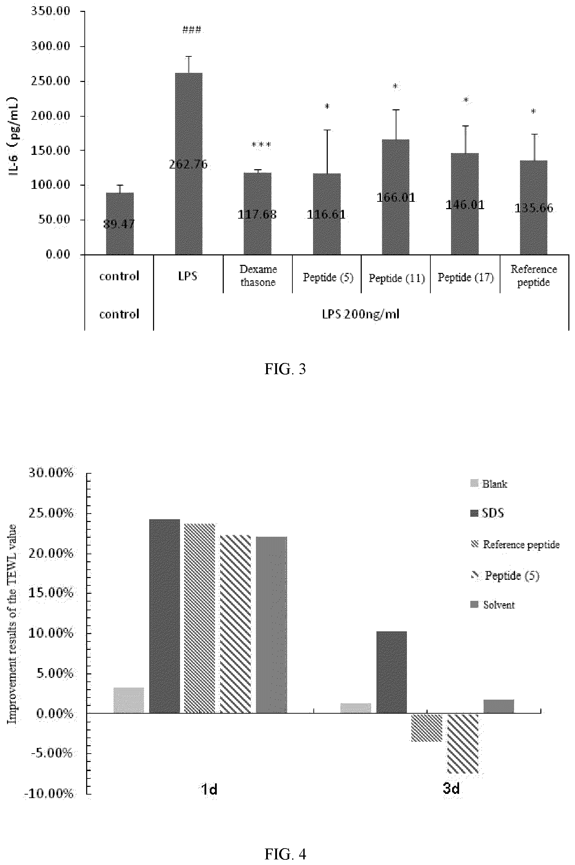

The experimental results are shown in FIGS. 2 and 3. After LPS stimulation, TNF-α and IL-6 cytokines were significantly up-regulated. After administration, the peptide (5), the peptide (11) as a comparative peptide, the peptide (17) as a comparative peptide, and the reference peptide at 100 ppm significantly down-regulated the TNF-α expression, having effects equivalent to that of the positive drug dexamethasone, or having even more excellent effects than dexamethasone; wherein the peptide (5) had a stronger effect on inhibiting the TNF-α expression. The peptide (5), the peptide (11) as a comparative peptide, the peptide (17) as a comparative peptide, and the reference peptide at 100 ppm also significantly down-regulated the expression of IL-6.

It is indicated that the peptides of the present invention have obvious anti-inflammatory and repairing effects.

### EXAMPLE 7 Damaged skin barrier repair experiment

### 7.1 Volunteers

### 7.1.1 Inclusion criteria:

Volunteers between the ages of 18 and 60 are eligible for the experiment requirements.

### 7.1.2 Rejection and shedding criteria:

Those who fail to use the test product as required and cannot determine the efficacy;
those who withdraw on their own initiative or fail to complete the prescribed observation period, thereby affecting the determination of efficacy;
those who have been enrolled but cannot continue to participate in the trial due to adverse events.

### 7.2 Reagents and instruments

Sodium dodecyl sulfate (SDS), a Finn chamber patch test device (a side length of 8 mm, an area of 64 mm²), a transepidermal water diffusion tester, and a C-Cube skin imaging system.

### 7.3 Sample preparation and grouping

Blank control: no substance added.
SDS group: 10% SDS solution.
Solvent group: 0.3% carbomer 980, 0.3% arginine, and the balance water. Preparation process: carbomer was dispersed in water, heated to 80°C, stirred and dissolved completely; the temperature was lowered to about 45°C; arginine was added, stirred, mixed and dissolved.

The 50 ppm reference peptide group: On the basis of the solvent group, the reference peptide was added, mixed and dissolved until the final concentration was 50 ppm.

The 50 ppm peptide (5) group: On the basis of the solvent group, the peptide (5) was added, mixed and dissolved until the final concentration was 50 ppm.

### 7.4 Determination steps

### 7.4.1 Establishment of damaged skin barrier

25 µL of 10% SDS solution was injected into a patch tester chamber to infiltrate a filter paper. Chambers were applied to the curved side of a subject's left arm with a hypoallergenic adhesive tape. The distance between the edges of adjacent patch tester chambers was not less than 3 cm. The chambers were pressed lightly with the palm of the subject's hand to apply them evenly on the skin for 6-8 h. After removing the patch tester, the subjects sat quietly in a standard testing environment for 30 min (after the indentation disappeared). The TEWL values were measured at the center of each patch area. Each area was measured twice. The measurement results were represented by an average value of TEWL values in 10 patch areas of each arm as the initial value (D0). During the sitting period, the subjects were not allowed to drink water or beverages. The forearms were exposed and placed in a test position while remaining relaxed, and the patch areas should be avoided from touch.

### 7.4.2 TEWL determination

After the initial TEWL value measurement was completed, the post-administration test was performed. Before each measurement, the test sites were cleaned with clean water and dried with a lint-free water absorbent paper towel. The subjects sat quietly for at least 20 min in a standard testing environment and could not drink water or beverages. The forearms were exposed and remained relaxed while the patch areas were avoided from touch. At the set measurement time point, the TEWL values were measured at the center of each patch area. Each area was measured twice. The measurement results were represented by average values of TEWL values in 10 patch areas on a single arm. The set measurement time interval was no less than 1 day, and the instrument test time should be set at D1 and D3 after modeling.

### 7.4.3 C-Cube imaging

The C-Cube was used to take pictures of the damaged area and record the erythema index.

### 7.5 Test evaluation

The test indicators were TEWL (Transepidermal Water Loss) and C-Cube 2D image analysis. Wherein, a TEWL value was obtained by measuring the partial pressure gradient of water vapor at different points near the epidermis (within 1 cm) per unit time and unit cross-sectional area under specified conditions of temperature and humidity, based on the Fick's law of diffusion. The higher the TEWL value, the more the transepidermal water loss per unit time and unit cross-sectional area, and the more serious the skin barrier is damaged; the lower the TEWL value, the less the transepidermal water loss per unit time and unit cross-sectional area, and the more minor the skin barrier is damaged.

The TEWL values measured on the 1st and 3rd days of administration were compared with the initial value (D0), respectively. The improvement results of the TEWL values are shown in FIG. 4. The results showed that after SDS stimulation, compared with the blank group, the TEWL values of the other groups significantly increased when tested at the time point on the 1st day. At the time point of 3rd day, compared with the SDS group, the TEWL of the peptide (5) significantly reduced, while the TEWL improvement value of the solvent group was not obvious on day 3 compared with day 0. It is indicated that the peptide (5) can reduce the TEWL value after SDS stimulation, repair the skin barrier after SDS stimulation, and has a better effect on skin barrier repair than the reference peptide.

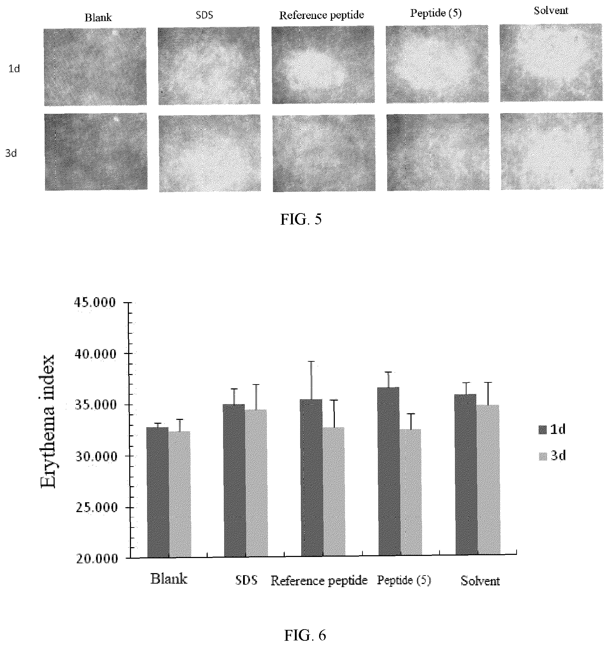

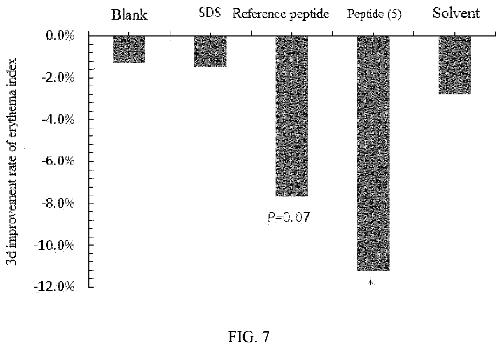

The erythema index can intuitively reflect the state of skin damage. The larger the value, the more serious the skin damage. FIG. 5 relates to photos showing the skin imaging results tested on the 1st and 3rd days after SDS stimulation. The results showed that the skin erythema area increased significantly after SDS stimulation. On the 3rd day, compared with the SDS group, the peptide (5) could significantly improve the erythema area. The results of the erythema index analysis are shown in FIGS. 6 and 7. The results showed that compared with the SDS group, the peptide (5) significantly reduced the erythema index, and the improvement value of the erythema index by the peptide (5) was better than that of the reference peptide.

It is indicated that the peptide (5) can repair the damaged skin and has an excellent effect on repairing the skin barrier.

### EXAMPLE 8 Preparation of a skin toner comprising the peptide (5)

| Ingredients | by weight % |
|---|---|
| Propylene glycol | 1.0 |
| Allantoin | 0.3 |
| Glycerin | 1.0 |
| PEG-7 glyceryl cocoate | 1.0 |
| Peptide (5) | 0.005 |
| Preservative | 0.5 |
| Perfume | 0.5 |
| Water | Balance to 100 |

Allantoin and glycerin were dissolved in water and heated to 85°C at which the temperature was kept for 30 min; PEG-7 glyceryl cocoate and the peptide (5) were dissolved in water; the above solutions were mixed after cooling, and stirred evenly to afford a mixed solution; propylene glycol, a preservative, and perfume were added to the above mixed solution in turn, and water was added with stirring evenly to afford a skin toner.

### EXAMPLE 9 Preparation of a cleansing gel comprising peptide (11) not according to the present invention

| Ingredients | by weight % |
|---|---|
| Carbomer (2% emulsion) | 25.0 |
| Triethanolamine | 0.5 |
| Peptide (11) | 0.005 |
| Preservative | 0.2 |
| EDTA | 0.1 |
| Perfume | 0.5 |
| Water | Balance to 100 |

The peptide (11) was dissolved in water and stirred evenly to afford a polypeptide solution; carbomer and EDTA were dissolved in water and heated to 85°C; the temperature was kept for 30 min; after cooling, triethanolamine was added and stirred evenly to afford a mixed solution; the above mixed solution, the polypeptide solution, a preservative, and perfume were added to water in turn and stirred until completely swollen to afford a cleansing gel.

### EXAMPLE 10 An emulsion composition comprising peptide (17) not according to the present invention

| Ingredients | by weight % |
|---|---|
| A cetearyl alcohol (and) cetearyl glucoside | 5.0 |
| Jojoba oil | 5.0 |
| Mineral oil | 5.0 |
| Isopropyl palmitate | 7.0 |
| B Glycerin | 5.0 |
| Allantoin | 0.1 |
| Polyacrylamide (and) C13-14 isoparaffin (and) laureth-7 | 0.3 |
| Water | Balance to 100 |
| C Preservative | 0.5 |
| D perfume | 0.5 |
| Peptide (17) | 0.01 |

0.01 g of the peptide (17) was formulated into a 0.02 mg/mL aqueous solution; cetearyl alcohol (and) cetearyl glucoside, jojoba oil, mineral oil, and isopropyl palmitate were heated to 85°C, and stirred evenly to afford phase A; glycerin, allantoin, polyacrylamide (and) C13-14 isoparaffin (and) laureth-7 were dissolved in water, and heated to 85°C to afford phase B; the phase A was quickly added into the phase B, homogenized at a constant temperature for 3-5 min and cooled; when the above mixture was cooled to below 60°C, a preservative was added and stirred evenly; when the above mixture was cooled to below 45°C, the polypeptide solution and perfume were added to afford an emulsion.

The above contents are further detailed descriptions of the present invention in conjunction with specific preferred embodiments and comparative data, but it is not indicated that the specific embodiments of the present invention are limited to these descriptions. For a person of ordinary skill in the art of the present invention, without departing from the concept of the present invention, some simple deductions or substitutions to be made should be regarded as belonging to the protection scope of the present invention.

## Claims

1. A peptide represented by Formula (I), or a stereoisomer thereof, or a mixture of stereoisomers thereof, or a cosmetically acceptable salt thereof, or a pharmaceutically acceptable salt thereof,
R₁-Phe-Phe-Trp-Phe-X₁-X₂-R₂ (I)
in Formula (I),
X₁-X₂ is -Lys-Val-;
R₁ is selected from H or R₃-CO-, wherein R₃ is selected from substituted or unsubstituted alkyl, substituted or unsubstituted alkenyl;
R₂ is selected from -NR₄R₅ or -OR₄, wherein each R₄ and R₅ is independently selected from H, substituted or unsubstituted alkyl, substituted or unsubstituted alkenyl;
the alkyl refers to a saturated aliphatic linear or branched chain alkyl having 1-24 carbon atoms (optionally having 1-16 carbon atoms; optionally having 1-14 carbon atoms; optionally having 1-12 carbon atoms; or optionally having 1, 2, 3, 4, 5, or 6 carbon atoms); optionally selected from methyl, ethyl, isopropyl, isobutyl, tert-butyl, pentyl, hexyl, heptyl, octyl, decyl, dodecyl, tetradecyl, hexadecyl, octadecyl, 2-ethylhexyl, 2-methylbutyl, or 5-methylhexyl;
the alkenyl refers to a linear or branched chain alkenyl having 2-24 carbon atoms (optionally having 2-16 carbon atoms; optionally having 2-14 carbon atoms; optionally having 2-12 carbon atoms; optionally having 2, 3, 4, 5, or 6 carbon atoms); the alkenyl has one or more carbon-carbon double bonds, optionally having 1, 2, or 3 conjugated or non-conjugated carbon-carbon double bonds; the alkenyl is bonded to the rest portions of a molecule through a single bond, and is optionally selected from vinyl, oleyl, or linoleyl;
optionally, the substituent in the "substituted alkyl" or "substituted alkenyl" is selected from C₁-C₄ alkyl; hydroxyl; C₁-C₄ alkoxy; amino; C₁-C₄ aminoalkyl; C₁-C₄ carbonyloxy; C₁-C₄ oxycarbonyl; halogen (such as fluorine, chlorine, bromine, and iodine); cyano; nitro; azide; C₁-C₄ alkylsulfonyl; thiol; C₁-C₄ alkylthio; C₆-C₃₀ aryloxy such as phenoxy; -NR_{b}(C=NR_{b})NR_{b}R_{c}, wherein R_{b} and R_{c} are independently selected from H, C₁-C₄ alkyl, C₂-C₄ alkenyl, C₂-C₄ alkynyl, C₃-C₁₀ cycloalkyl, C₆-C₁₈ aryl, C₇-C₁₇ aralkyl, a heterocyclic group having 3 to 10 members, or an amino protecting group.

2. The peptide represented by Formula (I) according to claim 1, or a stereoisomer thereof, or a mixture of stereoisomers thereof, or a cosmetically acceptable salt thereof, or a pharmaceutically acceptable salt thereof, **characterized in that** R₁ is selected from H, acetyl, *tert-*butyryl, hexanoyl, 2-methylhexanoyl, capryloyl, decanoyl, lauroyl, myristoyl, palmitoyl, stearoyl, oleoyl or linoleoyl;
optionally, R₁ is selected from H, acetyl, lauroyl, myristoyl or palmitoyl;
optionally, R₁ is H, acetyl or palmitoyl.

3. The peptide represented by Formula (I) according to claim 1, or a stereoisomer thereof, or a mixture of stereoisomers thereof, or a cosmetically acceptable salt thereof, or a pharmaceutically acceptable salt thereof, **characterized in that** R₄ and R₅ are independently selected from H, methyl, ethyl, hexyl, dodecyl or hexadecyl;
optionally, R₄ is H and R₅ is selected from H, methyl, ethyl, hexyl, dodecyl or hexadecyl;
optionally, R₂ is -OH or -NH₂.

4. The peptide represented by Formula (I) according to any of claims 1-3, or a stereoisomer thereof, or a mixture of stereoisomers thereof, or a cosmetically acceptable salt thereof, or a pharmaceutically acceptable salt thereof, **characterized in that** it is selected from the following peptides (1)-(6):
(1) H-Phe-Phe-Trp-Phe-Lys-Val-OH;
(2) Ac-Phe-Phe-Trp-Phe-Lys-Val-OH;
(3) Palm-Phe-Phe-Trp-Phe-Lys-Val-OH;
(4) H-Phe-Phe-Trp-Phe-Lys-Val-NH₂;
(5) Ac-Phe-Phe-Trp-Phe-Lys-Val-NH₂;
(6) Palm-Phe-Phe-Trp-Phe-Lys-Val-NH₂.

5. The peptide represented by Formula (I) according to claim 1, or a stereoisomer thereof, or a mixture of stereoisomers thereof, or a cosmetically acceptable salt thereof, or a pharmaceutically acceptable salt thereof, **characterized in that**
the cosmetically acceptable salt or pharmaceutically acceptable salt includes a metal salt of the peptide represented by Formula (I), the metal includes lithium, sodium, potassium, calcium, magnesium, manganese, copper, zinc or aluminum;
optionally, the cosmetically acceptable salt or pharmaceutically acceptable salt includes a salt formed from the peptide represented by Formula (I) and an inorganic acid or an organic acid, wherein the organic acid includes acetic acid, citric acid, lactic acid, malonic acid, maleic acid, tartaric acid, fumaric acid, benzoic acid, aspartic acid, glutamic acid, succinic acid, oleic acid, trifluoroacetic acid, oxalic acid, pamoic acid, or gluconic acid;
optionally, the inorganic acid includes hydrochloric acid, sulfuric acid, boric acid, or carbonic acid.

6. A cosmetic or pharmaceutical composition, **characterized in that** it includes an effective amount of the peptide represented by Formula (I) according to any of claims 1-5, or a stereoisomer thereof, or a mixture of stereoisomers thereof, or a cosmetically acceptable salt thereof, or a pharmaceutically acceptable salt thereof, and at least one excipient and optionally a cosmetically or pharmaceutically acceptable adjuvant;
optionally, the adjuvant is selected from a collagen synthesis stimulator, an agent that modulates PGC-1α synthesis, an agent that modulates the activity of PPARγ, an agent that increases or decreases triglyceride content in adipocytes, an agent that stimulates or delays adipocyte differentiation, a lipolytic agent or an agent that stimulates lipolysis, a fat clearing agent, an adipogenic agent, an inhibitor against acetylcholine receptor aggregation, an agent that inhibits muscle contraction, an anticholinergic agent, an elastase inhibitor, a matrix metalloproteinase inhibitor, a melanin synthesis stimulator or inhibitor, a whitening or decoloring agent, a pigmentation promoter, a self-tanning agent, an antiaging agent, a NO-synthase inhibitor, a 5α-reductase inhibitor, an inhibitor against lysyl hydroxylase and/or prolyl hydroxylase, an antioxidant, a free radical scavenger and/or an anti-air pollution agent, a reactive carbonyl species scavenger, an anti-glycation agent, an antihistamine, an antiviral agent, an antiparasitic agent, an emulsifier, an emollient, an organic solvent, a liquid propellant, a skin conditioner, a humectant, a moisture retaining substance, an alpha-hydroxy acid, a beta-hydroxy acid, a moisturizer, an epidermal hydrolase, a vitamin, an amino acid, a protein, a pigment or colorant, a dye, a biopolymer, a gelling polymer, a thickener, a surfactant, a softener, an adhesive, a preservative, an anti-wrinkle agent, an agent that can reduce or treat under-eye bags, an exfoliant, a cuticle stripping agent, a keratolytic agent, an antimicrobial, an antifungal, a fungistat, a bactericide, a bacteriostat, an agent that stimulates the synthesis of dermal or epidermal macromolecules and/or inhibits or prevents the degradation thereof, an agent that stimulates elastin synthesis, an agent that stimulates decorin synthesis, an agent that stimulates laminin synthesis, an agent that stimulates defensin synthesis, an agent that stimulates chaperone protein synthesis, an agent that stimulates cAMP synthesis, a heat shock protein, an agent that stimulates HSP70 synthesis, an agent that stimulates the synthesis of a heat shock protein, an agent that stimulates the synthesis of hyaluronic acid, an agent that stimulates fibronectin synthesis, an agent that stimulates deacetylase synthesis, an agent that stimulates the synthesis of a lipid and a stratum corneum component, ceramide, a fatty acid, an agent that inhibits collagen degradation, an agent that inhibits elastin degradation, an agent that inhibits serine protease, an agent that stimulates fibroblast proliferation, an agent that stimulates keratinocyte proliferation, an agent that stimulates adipocyte proliferation, an agent that stimulates melanocyte proliferation, an agent that stimulates keratinocyte differentiation, an agent that inhibits acetylcholinesterase, a skin relaxant, an agent that stimulates glycosaminoglycan synthesis, an anti-hyperkeratosis agent, a comedolytic agent, an antipsoriatic agent, an anti-dermatitis agent, an anti-eczema agent, a DNA repair agent, a DNA protecting agent, a stabilizer, an antipruritic agent, an agent for the treatment and/or care of sensitive skin, a hardening agent, a tightening agent, a restructuring agent, an anti-stretching agent, an adhesive, an agent that modulates sebum production, an antiperspirant, an agent that stimulates healing, an agent that assists healing, an agent that stimulates re-epithelialization, an agent that assists re-epithelialization, a cytokine growth factor, a sedative, an anti-inflammatory agent, an anesthetic agent, an agent acting on capillary circulation and/or microcirculation, an agent that stimulates angiogenesis, an agent that inhibits vascular permeability, a venotonic agent, an agent acting on cell metabolism, an agent for improving dermis-epidermis connection, an agent that induces hair growth, a hair growth inhibiting or retarding agent, a fragrance, a chelating agent, a plant extract, an essential oil, a marine extract, a reagent derived from a biological fermentation process, an inorganic salt, a cell extract, a sunscreen, and an effective organic or inorganic photoprotectant against A and/or B UV rays, or mixtures thereof.

7. The cosmetic or pharmaceutical composition according to claim 6, **characterized in that** a preparation of the cosmetic or pharmaceutical composition is selected from a cream, an oil, milk, a balm, a foam, a lotion, a gel, a liniment, a sera, a soap, a shampoo, a hair conditioner, a serum, an ointment, a mousse, a pomade, a powder, a bar, a pencil, a spray, an aerosol, a capsule, a tablet, a granule, a chewing gum, a solution, a suspension, an emulsion, a syrup, an elixir, a polysaccharide film, a jelly or a gelatin;
optionally, the capsule includes a soft capsule, a hard capsule, optionally a gelatin capsule;
optionally, the tablet includes a sugar-coated tablet.

8. A cosmetically or pharmaceutically acceptable delivery system or sustained release system, **characterized in that** it comprises an effective amount of the peptide represented by Formula (I) according to any of claims 1-5, or a stereoisomer thereof, or a mixture of stereoisomers thereof, or a cosmetically acceptable salt thereof, or a pharmaceutically acceptable salt thereof, or the cosmetic or pharmaceutical composition according to claim 6 or 7;
the cosmetically or pharmaceutically acceptable delivery system or sustained release system is selected from a liposome, an oleosome, a nonionic surfactant liposome vesicle, an ethosome, a millicapsule, a microcapsule, a nanocapsule, a nanostructured lipid carrier, a sponge, a cyclodextrin, a lipoid vesicle, a micelle, a millisphere, a microsphere, a nanosphere, a liposphere, a microemulsion, a nanoemulsion, a milliparticle, a microparticle or a nanoparticle; or optionally a liposome or microemulsion; or optionally a water-in-oil microemulsion having an internal structure of reverse micelle.

9. The peptide represented by Formula (I) according to any of claims 1-5, or a stereoisomer thereof, or a mixture of stereoisomers thereof, or a cosmetically acceptable salt thereof, or a pharmaceutically acceptable salt thereof, or the cosmetic or pharmaceutical composition according to claim 6 or 7, or the cosmetically or pharmaceutically acceptable delivery system or sustained release system according to claim 8 for use in anti-inflammation.

10. The peptide represented by Formula (I) according to any of claims 1-5, or a stereoisomer thereof, or a mixture of stereoisomers thereof, or a cosmetically acceptable salt thereof, or a pharmaceutically acceptable salt thereof, or the cosmetic or pharmaceutical composition according to claim 6 or 7, or the cosmetically or pharmaceutically acceptable delivery system or sustained release system according to claim 8 for use in skin barrier repair.

## Patentansprüche

1. Durch Formel (I) dargestelltes Peptid, oder ein Stereoisomer davon oder ein Gemisch von Stereoisomeren davon oder ein kosmetisch verträgliches Salz davon oder ein pharmazeutisch verträgliches Salz davon,
R₁-Phe-Phe-Trp-Phe-X₁-X₂-R₂ (I)
in Formel (I),
X₁-X₂ ist -Lys-Val-;
R₁ aus H oder R₃-CO- ausgewählt ist, wobei R₃ aus substituiertem oder unsubstituiertem Alkyl, substituiertem oder unsubstituiertem Alkenyl ausgewählt ist;
R₂ aus -NR₄R₅ oder -OR₄ ausgewählt ist, wobei R₄ und R₅ jeweils unabhängig aus H, substituiertem oder unsubstituiertem Alkyl, substituiertem oder unsubstituiertem Alkenyl ausgewählt sind;
Alkyl bezieht sich auf ein ges ätigtes aliphatisches lineares oder verzweigtes Alkyl mit 1-24 Kohlenstoffatomen (gegebenenfalls mit 1-16 Kohlenstoffatomen; gegebenenfalls mit 1-14 Kohlenstoffatomen; gegebenenfalls mit 1-12 Kohlenstoffatomen; oder gegebenenfalls mit 1, 2, 3, 4, 5 oder 6 Kohlenstoffatomen); gegebenenfalls ausgewählt aus Methyl, Ethyl, Isopropyl, Isobutyl, *tert-*Butyl, Pentyl, Hexyl, Heptyl, Octyl, Decyl, Dodecyl, Tetradecyl, Hexadecyl, Octadecyl, 2-Ethylhexyl, 2-Methylbutyl oder 5-Methylhexyl;
Alkenyl bezieht sich auf ein lineares oder verzweigtes Alkenyl mit 2-24 Kohlenstoffatomen (gegebenenfalls mit 2-16 Kohlenstoffatomen; gegebenenfalls mit 2-14 Kohlenstoffatomen; gegebenenfalls mit 2-12 Kohlenstoffatomen; gegebenenfalls mit 2, 3, 4, 5 oder 6 Kohlenstoffatomen); Alkenyl weist eine oder mehrere Kohlenstoff-Kohlenstoff-Doppelbindungen auf, gegebenenfalls mit 1, 2 oder 3 konjugierten oder nicht konjugierten Kohlenstoff-Kohlenstoff-Doppelbindungen; Alkenyl ist über eine Einfachbindung an die restlichen Teile eines Moleküls gebunden und ist gegebenenfalls aus Vinyl, Oleyl oder Linoleyl ausgewählt;
gegebenenfalls ist der Substituent in "substituiertem Alkyl" oder "substituiertem Alkenyl" ausgewählt aus C₁-C₄-Alkyl; Hydroxyl; C₁-C₄-Alkoxy; Amino; C₁-C₄-Aminoalkyl; C₁-C₄-Carbonyloxy; C₁-C₄-Oxycarbonyl; Halogen (wie Fluor, Chlor, Brom und Iod); Cyano; Nitro; Azid; C₁-C₄-Alkylsulfonyl; Thiol; C₁-C₄-Alkylthio; C₆-C₃₀-Aryloxy wie Phenoxy; -NR_{b}(C=NR_{b})NR_{b}R_{c}, wobei R_{b} und R_{c} unabhängig aus H, C₁-C₄-Alkyl, C₂-C₄-Alkenyl, C₂-C₄-Alkinyl, C₃₋C₁₀-Cycloalkyl, C₆-C₁₈-Aryl, C₇-C₁₇-Aralkyl, einer heterocyclischen Gruppe mit 3 bis 10 Gliedern oder einer Aminoschutzgruppe ausgewählt sind.

2. Ein durch Formel (I) dargestelltes Peptid nach Anspruch 1, oder ein Stereoisomer davon oder ein Gemisch von Stereoisomeren davon oder ein kosmetisch verträgliches Salz davon oder ein pharmazeutisch verträgliches Salz davon, **dadurch gekennzeichnet, dass** R₁ aus H, Acetyl, *tert-*Butyryl, Hexanoyl, 2-Methylhexanoyl, Capryloyl, Decanoyl, Lauroyl, Myristoyl, Palmitoyl, Stearoyl, Oleoyl oder Linoleoyl ausgewählt ist;
gegebenenfalls ist R₁ aus H, Acetyl, Lauroyl, Myristoyl oder Palmitoyl ausgewählt;
gegebenenfalls ist R₁ H, Acetyl oder Palmitoyl.

3. Ein durch Formel (I) dargestelltes Peptid nach Anspruch 1, oder ein Stereoisomer davon oder ein Gemisch von Stereoisomeren davon oder ein kosmetisch verträgliches Salz davon oder ein pharmazeutisch verträgliches Salz davon, **dadurch gekennzeichnet, dass** R₄ und R₅ unabhängig aus H, Methyl, Ethyl, Hexyl, Dodecyl oder Hexadecyl ausgewählt sind;
gegebenenfalls ist R₄ H und R₅ ist aus H, Methyl, Ethyl, Hexyl, Dodecyl oder Hexadecyl ausgewählt;
gegebenenfalls ist R₂ -OH oder -NH₂.

4. Ein durch Formel (I) dargestelltes Peptid nach einem der Ansprüche 1 bis 3, oder ein Stereoisomer davon oder ein Gemisch von Stereoisomeren davon oder ein kosmetisch verträgliches Salz davon oder ein pharmazeutisch verträgliches Salz davon, **dadurch gekennzeichnet, dass** es aus den folgenden Peptiden (1)-(6) ausgewählt ist:
(1) H-Phe-Phe-Trp-Phe-Lys-Val-OH;
(2) Ac-Phe-Phe-Trp-Phe-Lys-Val-OH;
(3) Palm-Phe-Phe-Trp-Phe-Lys-Val-OH;
(4) H-Phe-Phe-Trp-Phe-Lys-Val-NH₂;
(5) Ac-Phe-Phe-Trp-Phe-Lys-Val-NH₂;
(6) Palm-Phe-Phe-Trp-Phe-Lys-Val-NH₂.

5. Ein durch Formel (I) dargestelltes Peptid nach Anspruch 1, oder ein Stereoisomer davon oder ein Gemisch von Stereoisomeren davon oder ein kosmetisch verträgliches Salz davon oder ein pharmazeutisch verträgliches Salz davon, **dadurch gekennzeichnet, dass**
das kosmetisch verträliche Salz oder pharmazeutisch verträliche Salz ein Metallsalz des durch Formel (I) dargestellten Peptids einschließt, wobei das Metall Lithium, Natrium, Kalium, Calcium, Magnesium, Mangan, Kupfer, Zink oder Aluminium einschließt;
gegebenenfalls schließt das kosmetisch verträgliche Salz oder pharmazeutisch verträgliche Salz ein Salz ein, das aus dem durch Formel (I) dargestellten Peptid und einer anorganischen Säure oder einer organischen Säure gebildet ist, wobei die organische Säure Essigsäure, Citronensäure, Milchsäure, Malonsäure, Maleinsäure, Weins äure, Fumarsäure, Benzoesäure, Asparaginsäure, Glutaminsäure, Bernsteinsäure, Ölsäure, Trifluoressigsäure, Oxalsäure, Pamoasäure oder Gluconsäure einschließt;
gegebenenfalls schließt die anorganische Säure Salzsäure, Schwefelsäure, Borsäure oder Kohlensäure ein.

6. Kosmetische oder pharmazeutische Zusammensetzung, **dadurch gekennzeichnet, dass** sie eine wirksame Menge des durch Formel (I) dargestellten Peptids nach einem der Ansprüche 1 bis 5 oder eines Stereoisomers davon oder eines Gemischs von Stereoisomeren davon oder eines kosmetisch verträgichen Salzes davon oder eines pharmazeutisch verträglichen Salzes davon und mindestens einen Hilfsstoff und gegebenenfalls ein kosmetisch oder pharmazeutisch vertrißliches Adjuvans einschließt;
gegebenenfalls ist das Adjuvans ausgewählt aus einem Kollagensynthesestimulator, einem Mittel, das die PGC-1α-Synthese moduliert, einem Mittel, das die Aktivitä von PPARγ moduliert, einem Mittel, das den Triglyceridgehalt in Adipozyten erhöht oder verringert, einem Mittel, das die Adipozytendifferenzierung stimuliert oder verzögert, einem lipolytischen Mittel oder einem Mittel, das die Lipolyse stimuliert, einem Fettabbaumittel, einem adipogenen Mittel, einem Inhibitor gegen Acetylcholinrezeptoraggregation, einem Mittel, das die Muskelkontraktion hemmt, einem anticholinergen Mittel, einem Elastaseinhibitor, einem Matrixmetalloproteinaseinhibitor, einem Melaninsynthesestimulator oder -inhibitor, einem Aufhellungs- oder Entfärbungsmittel, einem Pigmentierungsförderer, einem Selbstbräunungsmittel, einem Anti-Aging-Mittel, einem NO-Synthase-Inhibitor, einem 5α-Reduktase-Inhibitor, einem Inhibitor gegen Lysylhydroxylase und/oder Prolylhydroxylase, einem Antioxidans, einem Radikalfänger und/oder einem Anti-Luftverschmutzungsmittel, einem Fänger reaktiver Carbonylspezies, einem Antiglykationsmittel, einem Antihistaminikum, einem antiviralen Mittel, einem antiparasitären Mittel, einem Emulgator, einem Emolliens, einem organischen Lösungsmittel, einem fllüssigen Treibmittel, einem Hautkonditionierungsmittel, einem Feuchthaltemittel, einer feuchtigkeitsbindenden Substanz, einer Alpha-Hydroxysäure, einer Beta-Hydroxysäure, einem Feuchtigkeitsmittel, einer epidermalen Hydrolase, einem Vitamin, einer Aminosäure, einem Protein, einem Pigment oder Farbmittel, einem Farbstoff, einem Biopolymer, einem gelbildenden Polymer, einem Verdickungsmittel, einem Tensid, einem Weichmacher, einem Haftmittel, einem Konservierungsmittel, einem Anti-Falten-Mittel, einem Mittel, das Tränensäcke reduzieren oder behandeln kann, einem Exfoliens, einem Mittel zum Ablösen der Kutikula, einem keratolytischen Mittel, einem antimikrobiellen Mittel, einem Antimykotikum, einem Fungistatikum, einem Bakterizid, einem Bakteriostatikum, einem Mittel, das die Synthese dermaler oder epidermaler Makromoleküle stimuliert und/oder deren Abbau hemmt oder verhindert, einem Mittel, das die Elastinsynthese stimuliert, einem Mittel, das die Decorinsynthese stimuliert, einem Mittel, das die Lamininsynthese stimuliert, einem Mittel, das die Defensinsynthese stimuliert, einem Mittel, das die Chaperonproteinsynthese stimuliert, einem Mittel, das die cAMP-Synthese stimuliert, einem Hitzeschockprotein, einem Mittel, das die HSP70-Synthese stimuliert, einem Mittel, das die Synthese eines Hitzeschockproteins stimuliert, einem Mittel, das die Synthese von Hyaluronsäure stimuliert, einem Mittel, das die Fibronectinsynthese stimuliert, einem Mittel, das die Deacetylasesynthese stimuliert, einem Mittel, das die Synthese eines Lipids und eines Bestandteils des Stratum corneum stimuliert, einem Ceramid, einer Fettsäure, einem Mittel, das den Kollagenabbau hemmt, einem Mittel, das den Elastinabbau hemmt, einem Mittel, das Serinprotease hemmt, einem Mittel, das die Fibroblastenproliferation stimuliert, einem Mittel, das die Keratinozytenproliferation stimuliert, einem Mittel, das die Adipozytenproliferation stimuliert, einem Mittel, das die Melanozytenproliferation stimuliert, einem Mittel, das die Keratinozytendifferenzierung stimuliert, einem Mittel, das Acetylcholinesterase hemmt, einem Hautrelaxans, einem Mittel, das die Glykosaminoglykansynthese stimuliert, einem Mittel gegen Hyperkeratose, einem komedolytischen Mittel, einem Antipsoriatikum, einem Mittel gegen Dermatitis, einem Mittel gegen Ekzem, einem DNA-Reparaturmittel, einem DNA-Schutzmittel, einem Stabilisator, einem juckreizstillenden Mittel, einem Mittel zur Behandlung und/oder Pflege empfindlicher Haut, einem H ätungsmittel, einem Straffungsmittel, einem Restrukturierungsmittel, einem Anti-Dehnungs-Mittel, einem Haftmittel, einem Mittel, das die Talgproduktion moduliert, einem Antitranspirant, einem Mittel, das die Heilung stimuliert, einem Mittel, das die Heilung unterst ützt, einem Mittel, das die Reepithelisierung stimuliert, einem Mittel, das die Reepithelisierung unterstützt, einem Zytokin-Wachstumsfaktor, einem Beruhigungsmittel, einem entzündungshemmenden Mittel, einem An ithetikum, einem Mittel, das auf die Kapillardurchblutung und/oder Mikrozirkulation wirkt, einem Mittel, das die Angiogenese stimuliert, einem Mittel, das die Gefäßpermeabilität hemmt, einem venentonisierenden Mittel, einem Mittel, das auf den Zellstoffwechsel wirkt, einem Mittel zur Verbesserung der Dermis-Epidermis-Verbindung, einem Mittel, das Haarwachstum induziert, einem Mittel, das Haarwachstum hemmt oder verzögert, einem Duftstoff, einem Chelatbildner, einem Pflanzenextrakt, einem äherischen Öl, einem Meeresextrakt, einem aus einem biologischen Fermentationsprozess stammenden Reagenz, einem anorganischen Salz, einem Zellextrakt, einem Sonnenschutzmittel und einem wirksamen organischen oder anorganischen Lichtschutzmittel gegen A- und/oder B-UV-Strahlen oder Gemischen davon.

7. Kosmetische oder pharmazeutische Zusammensetzung nach Anspruch 6, **dadurch gekennzeichnet, dass** eine Zubereitung der kosmetischen oder pharmazeutischen Zusammensetzung ausgewählt ist aus einer Creme, einem Öl, einer Milch, einem Balsam, einem Schaum, einer Lotion, einem Gel, einem Liniment, einem Serum, einer Seife, einem Shampoo, einem Haarconditioner, einem Serum, einer Salbe, einer Mousse, einer Pomade, einem Pulver, einem Riegel, einem Stift, einem Spray, einem Aerosol, einer Kapsel, einer Tablette, einem Granulat, einem Kaugummi, einer Lösung, einer Suspension, einer Emulsion, einem Sirup, einem Elixier, einem Polysaccharidfilm, einem Gelee oder einer Gelatine;
gegebenenfalls schließt die Kapsel eine Weichkapsel, eine Hartkapsel, gegebenenfalls eine Gelatinekapsel ein;
gegebenenfalls schließt die Tablette eine zucker überzogene Tablette ein.

8. Kosmetisch oder pharmazeutisch verträgliches Abgabesystem oder System mit verzögerter Freisetzung, **dadurch gekennzeichnet, dass** es eine wirksame Menge des durch Formel (I) dargestellten Peptids nach einem der Ansprüche 1 bis 5 oder eines Stereoisomers davon oder eines Gemischs von Stereoisomeren davon oder eines kosmetisch verträglichen Salzes davon oder eines pharmazeutisch verträglichen Salzes davon oder die kosmetische oder pharmazeutische Zusammensetzung nach Anspruch 6 oder 7 umfasst;
das kosmetisch oder pharmazeutisch verträgliche Abgabesystem oder System mit verzögerter Freisetzung ist ausgewählt aus einem Liposom, einem Oleosom, einem nichtionischen Tensidliposomvesikel, einem Ethosom, einer Millikapsel, einer Mikrokapsel, einer Nanokapsel, einem nanostrukturierten Lipidträger, einem Schwamm, einem Cyclodextrin, einem Lipoidvesikel, einer Mizelle, einer Millisphäre, einer Mikrosphäre, einer Nanosphäre, einer Liposphäre, einer Mikroemulsion, einer Nanoemulsion, einem Millipartikel, einem Mikropartikel oder einem Nanopartikel; oder gegebenenfalls einem Liposom oder einer Mikroemulsion; oder gegebenenfalls einer Wasser-in-Öl-Mikroemulsion mit einer inneren Struktur einer inversen Mizelle.

9. Peptid, dargestellt durch Formel (I) nach einem der Ansprüche 1 bis 5, oder ein Stereoisomer davon oder ein Gemisch von Stereoisomeren davon oder ein kosmetisch verträgliches Salz davon oder ein pharmazeutisch verträgliches Salz davon oder die kosmetische oder pharmazeutische Zusammensetzung nach Anspruch 6 oder 7 oder das kosmetisch oder pharmazeutisch verträgliche Abgabesystem oder System mit verzägerter Freisetzung nach Anspruch 8 zur Verwendung zur Entzündungshemmung.

10. Peptid, dargestellt durch Formel (I) nach einem der Ansprüche 1 bis 5, oder ein Stereoisomer davon oder ein Gemisch von Stereoisomeren davon oder ein kosmetisch verträgliches Salz davon oder ein pharmazeutisch verträgliches Salz davon oder die kosmetische oder pharmazeutische Zusammensetzung nach Anspruch 6 oder 7 oder das kosmetisch oder pharmazeutisch verträgliche Abgabesystem oder System mit verzögerter Freisetzung nach Anspruch 8 zur Verwendung zur Reparatur der Hautbarriere.

## Revendications

1. Peptide représenté par la Formule (I), ou un stéréoisomère de celui-ci, ou un m âange de stéréoisomères de celui-ci, ou un sel cosmétiquement acceptable de celui-ci, ou un sel pharmaceutiquement acceptable de celui-ci,
R₁-Phe-Phe-Trp-Phe-X₁-X₂-R₂ (I)
dans la Formule (I),
X₁-X₂ est -Lys-Val- ;
R₁ est choisi parmi H ou R₃-CO-, dans lequel R₃ est choisi parmi un alkyle substituéou non-substitué, un alcényle substitué ou non-substitu é;
R₂ est choisi parmi -NR₄R₅ ou -OR₄, dans lequel chacun de R₄ et R₅ est indépendamment choisi parmi H, un alkyle substitué ou non-substitué, un alcényle substitué ou non-substitué;
l'alkyle désigne un alkyle à chaîne linéaire ou ramifiée aliphatique saturé présentant de 1-24 atomes de carbone (optionnellement présentant de 1-16 atomes de carbone ; optionnellement présentant 1-14 atomes de carbone ; optionnellement présentant 1-12 atomes de carbone ; ou optionnellement présentant 1, 2, 3, 4, 5 ou 6 atomes de carbone) ; optionnellement choisi parmi un m éthyle, un éthyle, un isopropyle, un isobutyle, un tert-butyle, un pentyle, un hexyle, un heptyle, un octyle, un décyle, un dod écyle, un tédradécyle, un hexadécyle, un octadécyle, un 2-éthylhexyle, un 2-méthylbutyle ou un 5-méthylhexyle ;
l'alcényle désigne un alcényle à chaîne linéaire ou ramifiée présentant 2-24 atomes de carbone (optionnellement présentant 2-16 atomes de carbone ; optionnellement présentant 2-14 atomes de carbone ; optionnellement présentant 2-12 atomes de carbone ; optionnellement présentant 2, 3, 4, 5 ou 6 atomes de carbone) ; l'alcényle présente une ou plusieurs liaison(s) double(s) carbone-carbone, optionnellement présentant 1, 2 ou 3 liaison(s) double(s) carbone-carbone conjugu é(s) ou non-conjuguée(s) ; l'alcényle est lié aux parties restantes d'une molécule à travers une liaison simple, et est optionnellement choisi parmi le vinyle, l'oléyle ou le linoléyle ;
optionnellement, le substituant dans « l'alkyle substitué » ou « l'alcényle substitué » est choisi parmi un alkyle en C₁-C₄ ; un hydroxyle ; un alcoxy en C₁-C₃₄ ; un amino ; un aminoalkyle en C₁-C₄ ; un carbonyloxy en C₁-C₄ ; un oxycarbonyle en C₁-C₄ ; un halogène (tel que le fluor, le chlore, le brome et l'iode) ; un cyano ; un nitro ; un azide ; un alkylsulfonyle en C₁-C₄ ; un thiol ; un alkylthio en C₁-C₄ ; un aryloxy en C₆-C₃₀ tel qu'un phénoxy ; un -NR_{b}(C=NR_{b})NR_{b}R_{c}, dans lequel R_{b} et R_{c} sont choisis indépendamment parmi H, un alkyle en C₁-C₄, un alcényle en C₂-C₄, un alcynyle en C₂-C₄, un cycloalkyle en C₃-C₁₀, un aryle en C₆-C₁₈, un aralkyle en C₇-C₁₇, un groupe hétérocyclique présentant 3 à 10 chan̂ons, ou un groupe protecteur amino.

2. Peptide représentépar la Formule (I) selon la revendication 1, ou un stéréoisomère de celui-ci, ou un mélange de stéréoisomères de celui-ci, ou un sel cosm étiquement acceptable de celui-ci, ou un sel pharmaceutiquement acceptable de celui-ci, caractéris éen ce que R₁ est choisi parmi H, un ac dyle, un tert-butyryle, un hexanoyle, un 2-m éthylhexanoyle, un capryloyle, un décanoyle, un lauroyle, un myristoyle, un palmitoyle, un stéaroyle, un oléoyle ou un linoléoyle ;
optionnellement, R₁ est choisi parmi H, un acétyle, un lauroyle, un myristoyle ou un palmitoyle ;
optionnellement, R₁ est H, un acétyle ou un palmitoyle.

3. Peptide représentépar la Formule (I) selon la revendication 1, ou un stéréoisomère de celui-ci, ou un m dange de stéréoisomères de celui-ci, ou un sel cosm étiquement acceptable de celui-ci, ou un sel pharmaceutiquement acceptable de celui-ci, **caractérisé en ce que** R₄ et R₅ sont choisis indépendamment parmi H, un m éthyle, un éthyle, un hexyle, un dodécyle ou un hexadécyle ;
optionnellement, R₄ est H et R₅ est choisi parmi H, un m éthyle, un éthyle, un hexyle, un dodécyle ou un hexadécyle ;
optionnellement, R₂ est -OH ou -NH₂.

4. Peptide représenté par la Formule (I) selon l'une des revendications 1-3, ou un stéréoisomère de celui-ci, ou un m dange de stéréoisomères de celui-ci, ou un sel cosm étiquement acceptable de celui-ci, ou un sel pharmaceutiquement acceptable de celui-ci, **caractérisé en ce qu'**il est choisi parmi les peptides (1)-(6) suivants :
(1) H-Phe-Phe-Trp-Phe-Lys-Val-OH ;
(2) Ac-Phe-Phe-Trp-Phe-Lys-Val-OH ;
(3) Palm-Phe-Phe-Trp-Phe-Lys-Val-OH ;
(4) H-Phe-Phe-Trp-Phe-Lys-Val-NH₂ ;
(5) Ac-Phe-Phe-Trp-Phe-Lys-Val-NH₂ ;
(6) Palm-Phe-Phe-Trp-Phe-Lys-Val-NH₂.

5. Peptide représent épar la Formule (I) selon la revendication 1, ou un stéréoisomère de celui-ci, ou un m dange de stéréoisomères de celui-ci, ou un sel cosm diquement acceptable de celui-ci, ou un sel pharmaceutiquement acceptable de celui-ci, **caractérisé en ce que**
le sel cosmétiquement acceptable ou le sel pharmaceutiquement acceptable comporte un sel m étallique du peptide repr ésent é par la Formule (I), le métal comporte du lithium, du sodium, du potassium, du calcium, du magnésium, du manganèse, du cuivre, du zinc ou de l'aluminium ;
optionnellement, le sel cosm étiquement acceptable ou le sel pharmaceutiquement acceptable comporte un sel formé àpartir du peptide représenté par la Formule (I) et un acide inorganique ou un acide organique, dans lequel l'acide organique comporte de l'acide acétique, de l'acide citrique, de l'acide lactique, de l'acide malonique, de l'acide maléique, de l'acide tartrique, de l'acide fumarique, de l'acide benzoïque, de l'acide aspartique, de l'acide glutamique, de l'acide succinique, de l'acide oléique, de l'acide trifluoroacétique, de l'acide oxalique, de l'acide pamoïque ou de l'acide gluconique ;
optionnellement, l'acide inorganique comporte de l'acide chlorhydrique, de l'acide sulfurique, de l'acide borique ou de l'acide carbonique.

6. Composition cosmétique ou pharmaceutique, **caractérisée en ce qu'**elle comporte une quantité efficace du peptide représenté par la Formule (I) selon l'une des revendications 1-5, ou un stéréoisomère de celui-ci, ou un m dange de stéréoisomères de celui-ci, ou un sel cosmétiquement acceptable de celui-ci, ou un sel pharmaceutiquement acceptable de celui-ci, et au moins un excipient et optionnellement un adjuvant cosmétiquement ou pharmaceutiquement acceptable ;
optionnellement, l'adjuvant est choisi parmi un stimulateur de synthèse de collagène, un agent qui module la synthèse de PGC-1α, un agent qui module l'activité de PPARγ, un agent qui augmente ou diminue une teneur en triglycérides dans les adipocytes, un agent qui stimule ou retarde la différenciation adipocytaire, un agent lipolytique ou un agent qui stimule la lipolyse, un agent de clarification de graisse, un agent adipogène, un inhibiteur contre l'agrégation du récepteur de l'acétylcholine, un agent qui inhibe la contraction musculaire, un agent anticholinergique, un inhibiteur d'élastase, un inhibiteur de métalloprotéinases matricielles, un stimulateur ou un inhibiteur de synthèse de mélanine, un agent de blanchiment ou de décoloration, un promoteur de pigmentation, un agent autobronzant, un agent antivieillissement, un inhibiteur de NO-synthase, un inhibiteur de 5α-réductase, un inhibiteur contre la lysyl hydroxylase et/ou la prolyl hydroxylase, un antioxydant, un pi égeur de radicaux libres et/ou un agent anti-pollution de l'air, un piégeur d'espèces carbonyle réactives, un agent anti-glycation, un antihistaminique, un agent antiviral, un agent antiparasitaire, un émulsifiant, un émollient, un solvant organique, un propulseur liquide, un conditionneur de peau, un humectant, une substance retenant l'humidité, un acide alpha-hydroxy, un acide bêta-hydroxy, un hydratant, une hydrolase épidermique, une vitamine, un acide aminé, une protéine, un pigment ou un colorant, une teinture, un biopolymère, un polymère g difiant, un épaississant, un tensioactif, un adoucissant, un adhésif, un conservateur, un agent anti-rides, un agent qui peut réduire ou traiter des poches sous les yeux, un exfoliant, un agent de décapage de cuticule, un agent kératolytique, un antimicrobien, un antifongique, un fongistatique, un bactéricide, un bactériostatique, un agent qui stimule la synthèse de macromol éules dermiques ou épidermiques et/ou inhibe ou empêche la dégradation de celles-ci, un agent qui stimule la synthèse de l'élastine, un agent qui stimule la synthèse de la d éorine, un agent qui stimule la synthèse de la laminine, un agent qui stimule la synthèse de la défensine, un agent qui stimule la synthèse de la protéine chaperon, un agent qui stimule la synthèse de l'AMPc, une protéine de choc thermique, un agent qui stimule la synthèse de HSP70, un agent qui stimule la synthèse d'une protéine de choc thermique, un agent qui stimule la synthèse de l'acide hyaluronique, un agent qui stimule la synthèse de la fibronectine, un agent qui stimule la synthèse de la désacétylase, un agent qui stimule la synthèse d'un lipide et d'un composant de la couche cornée, un céramide, un acide gras, un agent qui inhibe la dégradation du collagène, un agent qui inhibe la dégradation de l'élastine, un agent qui inhibe la sérine protéase, un agent qui stimule la prolifération des fibroblastes, un agent qui stimule la prolifération des kératinocytes, un agent qui stimule la prolifération des adipocytes, un agent qui stimule la prolifération des mélanocytes, un agent qui stimule la différenciation des kératinocytes, un agent qui inhibe l'acétylcholinestérase, un relaxant de la peau, un agent qui stimule la synthèse des glycosaminoglycanes, un agent anti-hyperk éatose, un agent com édolytique, un agent anti-psoriasique, un agent anti-dermatite, un agent anti-eczéma, un agent de réparation d'ADN, un agent de protection d'ADN, un stabilisant, un agent antiprurigineux, un agent pour le traitement et/ou le soin de la peau sensible, un agent durcissant, un agent tenseur, un agent restructurant, un agent anti-vergetures, un adhésif, un agent qui module la production de sébum, un anti-transpirant, un agent qui stimule la cicatrisation, un agent qui aide àla cicatrisation, un agent qui stimule la ré-épith âialisation, un agent qui aide àla ré-épithélialisation, un facteur de croissance de cytokine, un sédatif, un agent anti-inflammatoire, un agent anesthésique, un agent agissant sur la circulation capillaire et/ou la microcirculation, un agent qui stimule l'angiogenèse, un agent qui inhibe la perméabilité vasculaire, un agent veinotonique, un agent agissant sur le métabolisme cellulaire, un agent d'amélioration de la liaison derme- épiderme, un agent qui induit la pousse des cheveux, un agent inhibant ou retardant la pousse des cheveux, un parfum, un agent chélatant, un extrait végétal, une huile essentielle, un extrait marin, un réactif dérivé d'un procédé de fermentation biologique, un sel inorganique, un extrait cellulaire, un écran solaire, et un photoprotecteur organique ou inorganique efficace contre les rayons UV A et/ou B, ou des m danges de ceux-ci.

7. Composition cosmétique ou pharmaceutique selon la revendication 6, **caractérisée en ce qu'**une préparation de la composition cosmétique ou pharmaceutique est choisie parmi une crème, une huile, un lait, un baume, une mousse coiffante, une lotion, un gel, un liniment, un sérum, un savon, un shampooing, un apr ès-shampooing, un sérum, un onguent, une mousse, une pommade, une poudre, une barre, un crayon, une pulvérisation, un aérosol, une capsule, un comprimé, un granulé, un chewing-gum, une solution, une suspension, une émulsion, un sirop, un élixir, un film de polysaccharide, une gelée ou une gélatine ;
optionnellement, la capsule comporte une capsule molle, une capsule dure, optionnellement une capsule de gélatine ;
optionnellement, le comprim écomporte un comprimé enrobé de sucre.

8. Système d'administration cosmétiquement ou pharmaceutiquement acceptable ou système à libération prolongée, **caractérisé en ce qu'**il comprend une quantité efficace du peptide repr ésent épar la Formule (I) selon l'une des revendications 1-5, ou un stéréoisomère de celui-ci, ou un mélange de stéréisomères de celui-ci, ou un sel cosmétiquement acceptable de celui-ci, ou un sel pharmaceutiquement acceptable de celui-ci, ou la composition cosmétique ou pharmaceutique selon la revendication 6 ou 7 ;
le système d'administration cosmétiquement ou pharmaceutiquement acceptable ou le système à libération prolongée est choisi parmi un liposome, un oléosome, une vésicule liposomale de tensioactif non-ionique, un éthosome, une millicapsule, une microcapsule, une nanocapsule, un vecteur lipidique nanostructuré, une éponge, une cyclodextrine, une vésicule lipoïdale, une micelle, une millisphère, une microsphère, une nanosph ère, une liposphère, une micro émulsion, une nanoémulsion, une milliparticule, une microparticule ou une nanoparticule ; ou optionnellement un liposome ou une microémulsion ; ou optionnellement une micro émulsion eau-dans-l'huile présentant une structure interne de micelle inverse.

9. Peptide représenté par la Formule (I) selon l'une des revendications 1-5, ou un stéréoisomère de celui-ci, ou un m dange de stéréoisomères de celui-ci, ou un sel cosmétiquement acceptable de celui-ci, ou un sel pharmaceutiquement acceptable de celui-ci, ou la composition cosmétique ou pharmaceutique selon la revendication 6 ou 7, ou le système d'administration cosmétiquement ou pharmaceutiquement acceptable ou le système à libération prolongée selon la revendication 8 pour son utilisation comme anti-inflammatoire.

10. Peptide représenté par la Formule (I) selon l'une des revendications 1-5, ou un stééoisomère de celui-ci, ou un m âange de stéréoisomères de celui-ci, ou un sel cosmétiquement acceptable de celui-ci, ou un sel pharmaceutiquement acceptable de celui-ci, ou la composition cosmétique ou pharmaceutique selon la revendication 6 ou 7, ou le système d'administration cosmétiquement ou pharmaceutiquement acceptable ou le système à libération prolongée selon la revendication 8 pour son utilisation dans la réparation de la barrière cutanée.
